# EUROPÄISCHE PATENTSCHRIFT

(11) **EP 0 847 976 B1**
(45) Veröffentlichungstag und Bekanntmachung des Hinweises auf die Patenterteilung: **27.06.2001**
(21) Anmeldenummer: 97121242.8
(22) Anmeldetag: 03.12.1997
(51) Int. Cl.: C07C 45/71, C07C 47/198

(54) **Verfahren zur Herstellung von Glyoxalmonoacetalen**
Process for the preparation of glyoxal monoacetals
Procédé pour la préparation de glyoxalmonoacétals

(30) Priorität: 11.12.1996 DE 19651325
(43) Veröffentlichungstag der Anmeldung: 17.06.1998
(73) Patentinhaber: BASF AKTIENGESELLSCHAFT, 67056 Ludwigshafen (DE)
(72) Erfinder: Gröning, Carsten, Dr., 68259 Mannheim (DE); Therre, Jörg, Dr., 67550 Worms (DE); Kaibel, Gerd, Dr., 68623 Lampertheim (DE); Ebel, Klaus, Dr., 68623 Lampertheim (DE)

(56) Entgegenhaltungen:
- EP-A- 0 249 530
- EP-A- 0 607 722
- SANGSARI F H ET AL: "The monoacetalization of glyoxal: a direct synthesis of 2,2-dimethoxy and diethoxyethanals" SYNTH. COMMUN. (SYNCAV,00397911);88; VOL.18 (12); PP.1343-8, LAB. CHIM. ORG. PHYS.;VILLEURBANNE; 69622; FR. (FR), XP002055556
- STAMBOULI A ET AL: "2,2-Dialkoxyethanals, bifunctional synthons with two carbons: preparation by acetalization of glyoxal and some synthetic applications" BULL. SOC. CHIM. FR. (BSCFAS,00378968);88; (1); PP.95-100, CNRS;LAB. CHIM. ORG. PHYS.; VILLEURBANNE; 69622; FR. (FR), XP002055557

## Beschreibung

Die vorliegende Erfindung betrifft ein neues Verfahren zur Herstellung von Glyoxalmonoacetalen.

Die Umsetzung von Glyoxal mit Alkoholen, z.B. mit Methanol, ist schon lange bekannt und u.a. in J. Org. Chem. 38, 556 (1973), J. Am. Chem. Soc. 77, 1285 (1955) sowie in der US-Patentschrift 2,360,959 beschrieben. Allerdings erhält man nach den obigen Literaturstellen lediglich die entsprechenden Glyoxalbisacetale; die Isolierung von Glyoxalmonoacetalen, beispielsweise Glyoxaldimethylacetal ist darin nicht offenbart.

Die gezielte Herstellung und Isolierung von Glyoxalmonoacetalen ist Gegenstand der Veröffentlichungen in Synth. Comm. 1343 (1988) und Bull. Soc. Chim. Fr. 95 (1988) sowie der EP-B-0 249 530, in denen man Glyoxal in Anwesenheit eines sauren Katalysators mit einem Überschuß eines Alkohols umsetzt und dann die Reaktion abbricht, sobald die Konzentration an gewünschtem Monoacetal im Reaktionsmedium zugunsten des Bisacetals nachläßt, wobei die Reaktion durch Analyse von regelmäßig aus dem Reaktionsmedium entnommenen Proben überwacht wird.

Diese Vorgehensweise hat jedoch den Nachteil, daß die Reaktion ständig per Gaschromatographie kontrolliert werden muß, die Destillation mittels Drehbandkolonne sehr aufwendig ist und das Nebenprodukt Glyoxalbisacetal, z.B. 1,1,2,2-Tetramethoxyethan, offenbar verworfen wird.

Daneben beschreibt EP-B-0 316672 ein Verfahren zur Herstellung von Monoacetalen des Glyoxals, das sich speziell auf die Umsetzung von Glyoxal mit substituiertem 1,3-Propandiol beschränkt und somit zu cyclischen Monoacetalen führt.

Es bestand daher die Aufgabe, ein Verfahren zur Herstellung von Glyoxalmonoacetalen vorzuschlagen, das die o.g. Nachteile nicht aufweist.

Es wurde nun gefunden, daß man Monoacetale des Glyoxals der allgemeinen Formel I, 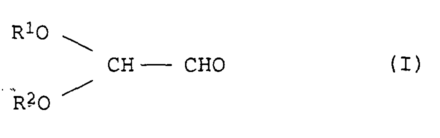 worin R¹ und R², die gleich oder verschieden sein können, eine C₁-C₄ Alkyl- oder C₂-C₄ Alkenylgruppe bedeuten, auf technisch besonders vorteilhafte Weise dadurch herstellen kann, daß man eine Mischung aus Glyoxal und Glyoxalbisacetalen der allgemeinen Formel II, 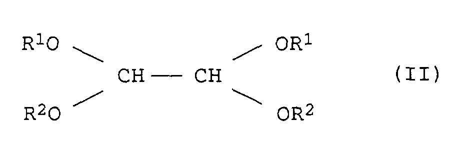 worin R¹ und R² die oben genannte Bedeutung haben, in Anwesenheit eines sauren Katalysators mit einem Überschuß eines Alkohols der allgemeinen Formel R¹OH und/oder R²OH bis zur Erreichung des Reaktionsgleichgewichts umsetzt.

Die Reste R¹ und R² der Formeln I und II leiten sich direkt vom eingesetzten Alkohol der allgemeinen Formel R¹OH und/oder R²OH ab und haben somit auch analoge Bedeutung. R¹ und R² stehen dabei für verzweigtes oder unverzweigtes C₁-C₄ Alkyl- und/oder für verzweigtes oder unverzweigtes C₂-C₄ Alkenyl. C₁-C₄ Alkyl bedeutet z.B. Methyl, Ethyl, Propyl, iso-Propyl und Butyl. C₂-C₄ Alkenyl bedeutet z.B. Vinyl, Propenyl und Isopropenyl. Besonders bevorzugte Reste für R¹ und R² sind Methyl und Ethyl.

Die erfindungsgemäße Acetalisierung von Glyoxal wird mit einem Überschuß von 5 bis 20 Mol, bevorzugt 10 bis 15 Mol Alkohol pro Mol Glyoxal durchgeführt.

Das eingesetzte Glyoxal liegt vorzugsweise in Form einer wässrigen Lösung vor, wobei man zweckmäßigerweise die üblichen technischen wässrigen Lösungen mit einem Glyoxalgehalt von 20 bis 60, vorzugsweise 30 bis 50 Gew.% verwendet. Es ist aber auch möglich, kristallines Glyoxal, als Trimeres mit zwei Mol Kristallwasser, für die Acetalisierung einzusetzen.

Die Menge an eingesetztem bzw. rückgeführtem Glyoxalbisacetal der Formel II hängt einerseits von der jeweiligen Konzentration der Reaktanten im Gleichgewichtszustand sowie andererseits von der erzielten Destillationsausbeute an Glyoxalbisacetal im Aufarbeitungsschritt ab. Im allgemeinen werden pro Mol Glyoxal 0,4 bis 1 Mol, insbesondere 0,4 bis 0,7 Mol des Glyoxalbisacetals der Formel II eingesetzt.

Das Verfahren kann bei Normaldruck, vermindertem oder erhöhtem Druck durchgeführt werden. In der Regel erfolgt die Reaktion bei Normaldruck bei der Siedetemperatur des Reaktionsgemisches oder im geschlossenen System bei, dem jeweiligen Eigendruck des Systems entsprechenden Siedetemperaturen.

Das neue Verfahren eignet sich sowohl für die kontinuierliche als auch für die diskontinuierliche Arbeitsweise, wobei als einsetzbare Reaktoren z.B. kontinuierlich oder diskontinuierlich betriebene Rührkessel, Rohrreaktoren und- Kolonnen in Betracht kommen.

Als saure Katalysatoren kommen sowohl Lewis- als auch BrönstedtSäuren in Frage. So können beispielsweise Zirkoniumsulfat sowie Schwefelsäure, Methansulfonsäure, p-Toluolsulfonsäure, Trichloressigsäure, Oxalsäure und bevorzugt saure Ionenaustauscher, insbesondere solche in makroporöser Form eingesetzt werden. Beispiele für makroporöse saure Ionenaustauscher sind u.a. die handelsüblichen Produkte Lewatit® S 100, BayKat® K 2611 (Bayer), Amberlite® IR-120 (Rohm & Haas) sowie Dowex® 50 (Dow Chemicals). In einer bevorzugten Verfahrensausführung wird der saure Ionenaustauscher in eine Säule als Festbett eingefüllt, durch das die Reaktionsmischung im Kreis gepumpt wird. Im allgemeinen verwendet man dabei den Katalysator in Mengen von 0,01 Mol bis 0,25 Mol pro Mol Glyoxal.

Anders als in dem in EP-B-0 249 530 beanspruchten Herstellprozeß, in dem die Acetalisierung vor der Gleichgewichtseinstellung im Maximum der Glyoxalmonoacetal-Bildung abgebrochen wird, wird im vorliegenden erfindungsgemäßen Verfahren die Reaktion bis zur Erreichung des Gleichgewichtszustands durchgeführt. Die Zeit, in der sich das Acetalisierungsgleichgewicht einstellt, läßt sich schnell in einem Vorversuch anhand einer Reaktionskinetik ermitteln und beträgt in der Regel unter den oben genannten Reaktionsbedingungen 3 bis 8 Stunden, bevorzugt 4 bis 6 Stunden.

Am Ende der Reaktion beziehungsweise nach Erreichen des Reaktionsgleichgewichts wird der saure Katalysator durch Neutralisation mit einer geeigneten Base wie z.B. Alkali- oder Erdalkalihydroxide oder Carbonate im Reaktionsgemisch desaktiviert oder im Falle eines Ionenaustauscherharzes durch Filtration aus dem Reaktionsgemisch entfernt um ihn im nächsten Reaktionsansatz wieder einsetzen zu können. Bei Verwendung eines Ionenaustauscherfestbetts wird dieses nach Beendigung der Reaktion einfach vom Umpumpkreislauf abgekoppelt.

Nach Deaktivierung bzw. Abtrennung des sauren Katalysators wird der überschüssige Alkohol abdestilliert und ebenfalls in den nächsten Reaktionszyklus zurückgeführt. Das gebildete Glyoxalbisacetal wird unter Zusatz von Wasser als Homoazeotrop entfernt. Dabei wird dem Reaktionsgemisch zur vollständigen Abtrennung des angefallenen Nebenprodukts soviel Wasser zugesetzt, daß das Bisacetal als 20 bis 50 Gew.-%ige, bevorzugt als 30 Gew.-%ige wässrige Lösung anfällt und das Glyoxalmonoacetal als 30 bis 50 Gew.-%ige, bevorzugt als 40 Gew.-%ige Roh-Lösung ausgeschleust werden kann. Für die Rückführung des Nebenproduktes Glyoxalbisacetal ist es vorteilhaft, das Glyoxalbisacetal/Wasser-Azeotrop zunächst zu entwässern. Dies kann in an sich bekannter Weise durch azeotrope Destillation in Gegenwart eines Schleppmittels, wie z.B. Hexan, Cyclohexan, Heptan, Octan, Toluol oder Xylol erfolgen.

Das ausgeschleuste Roh-Glyoxalmonoacetal kann durch eine Wasserdampf-Destillation und anschließende fraktionierte Destillation, gegebenenfalls unter vermindertem Druck aufgereinigt werden, wobei es vorteilhaft ist, zur Vermeidung von Zersetzung des Wertprodukts, den pH-Wert der Rohlösung vor der Destillation durch Zusatz einer Base, z.B. Na₂CO₃, auf Werte zwischen 6,5 und 8,5 einzustellen.

Zur Vervollständigung der Rückführkreisläufe konnte überraschenderweise auch der Rückstand der Wasserdampfdestillation im nachfolgenden Herstellprozeß erneut eingesetzt werden. Um eine Desaktivierung des sauren Katalysators zu vermeiden, ist in diesem Fall von einer Neutralisation des Destillationsrückstandes mit beispielsweise Na₂CO₃ abzusehen.

Bei dem erfindungsgemäßen Herstellverfahren für Glyoxalmonoacetalen erübrigt sich eine zum Teil aufwendig durchzuführende Reaktionsanalytik und durch die oben beschriebene Aufarbeitung, einschließlich der Rückführungen, kommen besonders umwelt- und ressourcenschonenden und damit auch wirtschaftliche Faktoren zum Tragen.

Im folgenden Beispiel wird die Durchführung des erfindungsgemäßen Verfahrens näher erläutert.

### Allgemeine Arbeitsvorschrift:

Eine Mischung aus 725 g einer 40 Gew.%igen wässrigen Glyoxallösung (5 mol), 2400 g (75 mol) Methanol und ca. 450 g (3 mol) Tetramethoxyethan (TME) wurde bei 70°C unter dem Eigendruck des Systems durch drei hintereinander geschaltete, mit saurem Ionenaustauscher (BayKat® K 2611) gefüllte Rohrreaktoren (Katalysatorvolumen: 1,14 1) mit einer Pumpleistung von 14-15 l/h im Kreis gepumpt. Nach 5 stündiger Reaktionszeit wurde das Gemisch mit 20 Gew.%iger, wässriger Na₂CO₃-Lösung neutralisiert und das überschüssige Methanol bei 350 mbar bis zu einem Rücklaufverhältnis von 10:1 und einer Kopftemperatur von 41°C über eine mit 19 Gewebepackungen (Fa. Sulzer) gefüllte Kolonne (Länge: 100 cm, Durchmesser 3 cm, Bodenzahl: ca. 20) abdestilliert. Das Methanol wurde nach gaschromatographischer Analytik mit frischem Methanol auf 75 mol ergänzt und in den nächsten Ansatz zurückgeführt.

Unter Zusatz von Wasser wurde anschließend Tetramethoxyethan als wässriges Azeotrop aus dem Reaktionsansatz bei 350 mbar und einer Kopftemperatur von 72°C über die oben genannte Destillationskolonne abdestilliert.

Das TME/Wasser-Azeotrop wurde mit 500 g Octan versetzt und bei 600 mbar destillativ entwässert. Das TME wurde über Sumpf ausgeschleust und ebenso wie die Octanphase zurückgeführt.

Der Glyoxaldimethylacetal-haltige Destillationsrückstand wurde nach der TME Abtrennung ausgeschleust und destillativ aufgereinigt.

Die folgende Tabelle faßt die Ergebnisse von fünf Versuchen einschließlich vier Rückführungen zusammen.

| | | | | | |
|---|---|---|---|---|---|
| Versuch | Rückführung | Glyoxal [mol] | TME [g ; mol] | Glyoxaldimethylacetal [g] | Roh-Ausbeute* [%] |
| 1 | | 5 | 450 ; 3 | 390 | 74,8 |
| 2 | 1. | 5 | 450*; 3 | 367 | 70,4 |
| 3 | 2. | 5 | 434*; 2,9 | 395 | 75,8 |
| 4 | 3. | 5 | 456* ; 3 | 407 | 78,2 |
| 5 | 4. | 5 | 464* ; 3,1 | 410 | 78,8 |
| | 5. | | * = rückge-führtes TME | | * bezogen auf eingesetztes Glyoxal |

## Patentansprüche

1. Verfahren zur Herstellung von Glyoxalmonoacetalen der allgemeinen Formel I, 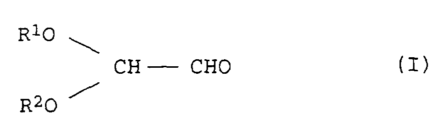 worin R¹ und R², die gleich oder verschieden sein können, eine C₁-C₄ Alkyl- oder C₂-C₄ Alkenylgruppe darstellen, dadurch gekennzeichnet, daß man eine Mischung aus Glyoxal und Glyoxalbisacetalen der allgemeinen Formel II, 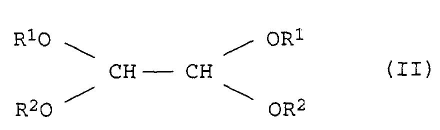 worin R¹ und R² die oben genannte Bedeutung haben, in Anwesenheit eines sauren Katalysators mit einem Überschuß von 5-20 Mol eines Alkohols R¹OH oder R²OH oder Gemischen davon, bis zur Erreichung des Reaktionsgleichgewichts umsetzt, wobei pro Mol Glyoxal 0,4 bis 1 Mol des Glyoxalbisacetals der Formel (II) eingesetzt werden.

2. Verfahren nach Anspruch 1 dadurch gekennzeichnet, daß der saure Katalysator ein saurer Ionenaustauscher ist.

3. Verfahren nach den Ansprüchen 1 und 2 dadurch gekennzeichnet, daß der saure Ionenaustauscher als Festbett vorliegt.

4. Verfahren nach den Ansprüchen 1 bis 3 dadurch gekennzeichnet, daß das Glyoxalbisacetal der allgemeinen Formel II als Homoazeotrop mit Wasser destillativ abgetrennt wird.

5. Verfahren nach Anspruch 1 bis 4 dadurch gekennzeichnet, daß das Azeotrop aus Glyoxalbisacetal der allgemeinen Formel II und Wasser mit einem geeigneten Schleppmittel entwässert wird.

6. Verfahren nach den Ansprüchen 1 bis 5 dadurch gekennzeichnet, daß das Nebenprodukt Glyoxalbisacetal der allgemeinen Formel II in die Reaktion zurückgeführt wird.

7. Verfahren nach den Ansprüchen 1 bis 6 dadurch gekennzeichnet, daß der saure Katalysator zurückgeführt wird.

8. Verfahren nach den Ansprüchen 1 bis 7 dadurch gekennzeichnet, daß der überschüssige Alkohol und der Destillationssumpf wieder in die Reaktion zurückgeführt werden.

9. Verfahren nach den Ansprüchen 1 bis 8 dadurch gekennzeichnet, daß der Alkohol der allgemeinen Formel R¹OH oder R²OH Methanol ist.

## Claims

1. A process for preparing glyoxal monoacetals of the formula I, 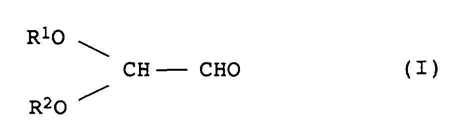 where R¹ and R², which can be identical or different, are C₁-C₄ alkyl or C₂-C₄ alkenyl, which comprises reacting a mixture of glyoxal and glyoxal bisacetals of the formula II, 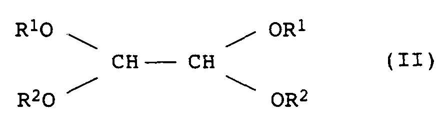 where R¹ and R² have the meaning given above, with an excess of 5-20 mol of an alcohol R¹OH or R²OH or mixtures of these in the presence of an acid catalyst until reaction equilibrium is achieved, from 0.4 to 1 mol of the glyoxal bisacetal of the formula (II) being used per mole of glyoxal.

2. A process as claimed in claim 1, wherein the acid catalyst is an acidic ion exchanger.

3. A process as claimed in claim 1 or 2, wherein the acidic ion exchanger is present as a fixed bed.

4. A process as claimed in any of claims 1 to 3, wherein the glyoxal bisacetal of the formula II is separated off by distillation as a homoazeotrope with water.

5. A process as claimed in any of claims 1 to 4, wherein the azeotrope of glyoxal bisacetal of the formula II and water is dehydrated with a suitable entrainer.

6. A process as claimed in any of claims 1 to 5, wherein the by-product glyoxal bisacetal of the formula II is recycled to the reaction.

7. A process as claimed in any of claims 1 to 6, wherein the acid catalyst is recycled.

8. A process as claimed in any of claims 1 to 7, wherein the excess alcohol and the distillation bottom phase are recycled back to the reaction.

9. A process as claimed in any of claims 1 to 8, wherein the alcohol of the formula R¹OH or R²OH is methanol.

## Revendications

1. Procédé pour la préparation de monoacétals de glyoxal de formule générale I, 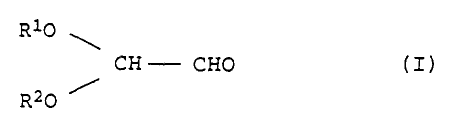 où R¹ et R², qui peuvent être identiques ou différents, représentent un groupe alkyle en C₁-C₄ ou un groupe alcényle en C₂-C₄, caractérisé par le fait qu'on fait réagir un mélange de glyoxal et de bisacétals de glyoxal de formule générale II, 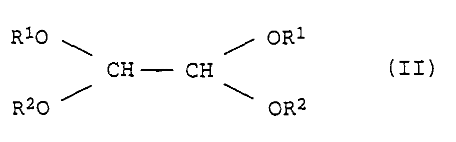 où R¹ et R² ont la signification indiquée plus haut, en présence d'un catalyseur acide avec un excès de 5-20 mol d'un alcool R¹OH ou R²OH ou d'un mélange de ceux-ci, jusqu'à ce qu'on atteigne l'équilibre réactionnel, tandis qu'on utilise par mol de glyoxal 0,4 à 1 mol de bisacétals de glyoxal de formule II.

2. Procédé selon la revendication 1, caractérisé par le fait que le catalyseur acide est un échangeur d'ions acide.

3. Procédé selon les revendications 1 et 2, caractérisé par le fait que l'échangeur d'ions acide est présent sous forme de lit solide.

4. Procédé selon les revendications 1 à 3, caractérisé par le fait que le bisacétal de glyoxal de formule générale II est éliminé par distillation sous forme d'homoazéotrope avec de l'eau.

5. Procédé selon la revendication 1 à 4, caractérisé par le fait que l'azéotrope de bisacétal de glyoxal de formule générale II et d'eau est déshydraté avec un agent d'entraînement approprié.

6. Procédé selon les revendications 1 à 5, caractérisé par le fait que le sous-produit de bisacétal de glyoxal de formule générale II est renvoyé dans la réaction.

7. Procédé selon les revendications 1 à 6, caractérisé par le fait que le catalyseur acide est recyclé.

8. Procédé selon les revendications 1 à 7, caractérisé par le fait que l'alcool en excès et la pâte de distillation sont envoyés à nouveau dans la réaction.

9. Procédé selon les revendications 1 à 8, caractérisé par le fait que l'alcool de formule générale R¹OH ou R²OH est le méthanol.
